# EUROPEAN PATENT SPECIFICATION

(11) **EP 3 958 758 B1**
(45) Date of publication and mention of the grant of the patent: **14.10.2026**
(21) Application number: 19925665.2
(22) Date of filing: 23.04.2019
(51) Int. Cl.: A61B 17/70, A61B 34/10, A61B 34/20, A61B 90/00, A61B 17/88

(54) **SURGICAL SYSTEM**
CHIRURGISCHES SYSTEM
SYSTÈME CHIRURGICAL

(43) Date of publication of application: 02.03.2022
(73) Proprietor: Warsaw Orthopedic, Inc., Warsaw, IN 46581 (US)
(72) Inventor: JOSSE, Loic, Collierville, Tennessee 38017 (US); PEULTIER, Bertrand, 25370 Les Hopitaux Neufs (FR); PREVOST, Julien J., Memphis, Tennessee 38117 (US); ELLIOTT, John A., Atoka, Tennessee 38004 (US); GRIZZARD, Mark R., Munford, Tennessee 38058 (US); BOBBITT, Dustin, Olive Branch, Mississippi 38654 (US)
(74) Representative: Viering, Jentschura & Partner mbB Patent- und Rechtsanwälte
(86) International application number: PCT/US2019/028612
(87) International publication number: WO 2020/219015

(56) References cited:
- WO-A1-2018/150214
- WO-A1-2018/150215
- KR-B1- 101 446 620
- US-A1- 2016 166 335
- US-A1- 2016 206 442
- US-A1- 2017 100 116
- US-A1- 2017 252 107
- US-A1- 2017 252 107
- US-A1- 2018 161 101
- US-A1- 2018 303 552
- US-A1- 2019 038 366
- US-A1- 2019 069 956

## Description

### TECHNICAL FIELD

The present disclosure generally relates to medical devices for the treatment of spinal disorders, and more particularly to a surgical system for correction of a spinal disorder.

### BACKGROUND

Spinal disorders such as degenerative disc disease, disc herniation, osteoporosis, spondylolisthesis, stenosis, scoliosis, kyphosis, and other curvature abnormalities, tumor, and fracture may result from factors including trauma, disease and degenerative conditions caused by injury and aging. Spinal disorders typically result in symptoms including pain, nerve damage, and partial or complete loss of mobility.

Non-surgical treatments, such as medication, rehabilitation and exercise can be effective, however, may fail to relieve the symptoms associated with these disorders. Surgical treatment of these spinal disorders includes correction, ligamentotaxy, corpectomy, discectomy, laminectomy, fusion, fixation and implantable prosthetics. Correction treatments used for positioning and alignment of vertebrae may employ spinal implants including spinal constructs and interbody devices for stabilization of a treated section of a spine. In some cases, the spinal implants may be manipulated with surgical instruments for compression and distraction of vertebrae. This disclosure describes an improvement over these prior technologies. From US 2018/303552 A1 a spinal implant system is known, wherein the spinal implant system comprises: a tangible storage device comprising computer-readable instructions; and a processor, executing the instructions in operation of the system for: determining, pre-operatively, a post-correction orientation of the patient anatomy according to the implant strategy and the manipulation strategy; and displaying, pre-operatively, the post-correction orientation of the patient anatomy, wherein the processor further executes the instructions in operation of the system for: comparing, intra-operatively, an actual correction orientation of the patient anatomy according to the manipulation strategy with the pre-operatively determined post-correction orientation, wherein the spinal implant system further comprises: at least one bone fastener having a screw shaft, wherein determining, pre-operatively, the implant strategy includes selecting a screw trajectory for the screw shaft of the bone fastener connecting the screw shaft to vertebral tissue. US2017/252107 A1, US2016/166335 A1 and US2019/038366 A1 disclose further spinal implant systems of the prior art.

### SUMMARY

The object of the present disclosure is providing an improved spinal implant system for the treatment of spinal disorders. This object is solved by a system according to claim 1. Further embodiments are subject of the dependent claims.

### BRIEF DESCRIPTION OF THE DRAWINGS

The present disclosure will become more readily apparent from the specific description accompanied by the following drawings, in which:
FIG. 1 is a perspective view of components of one embodiment of a surgical system in accordance with the principles of the present disclosure disposed with vertebrae;
FIG. 2 is a perspective view of components of one embodiment of a surgical system in accordance with the principles of the present disclosure disposed with vertebrae;
FIG. 3 is a perspective view of components, disposed with vertebrae, of one embodiment of a surgical system in accordance with the principles of the present disclosure;
FIGS. 4 and 5 are flow diagrams illustrating representative steps of embodiments of a surgical system in accordance with the principles of the present disclosure;
FIG. 6 is a perspective view of components of one embodiment of a surgical system in accordance with the principles of the present disclosure disposed with vertebrae;
FIGS. 7 is a plan view of one embodiment of an implant strategy in accordance with the principles of the present disclosure;
FIGS. 8 and 9 are graphical representations of a computer showing components of one embodiment of a spinal implant system in accordance with the principles of the present disclosure;
FIG. 10 is a perspective view of one embodiment of a manipulation strategy in accordance with the principles of the present disclosure;
FIG. 11 is a side view of one embodiment of a manipulation strategy in accordance with the principles of the present disclosure; and
FIG. 12 is a side view of one embodiment of a manipulation strategy in accordance with the principles of the present disclosure.

### DETAILED DESCRIPTION

The exemplary embodiments of the system and related methods of use disclosed are discussed in terms of medical devices for the treatment of musculoskeletal disorders and more particularly, in terms of a surgical system and method for correction of a spine disorder. In some embodiments, the present surgical system includes one or more surgical instruments that allow vertebral manipulation to treat spinal disorders, as described herein, for managing lordosis and/or kyphosis restoration. In some embodiments, the present surgical system includes one or more surgical instruments that allow for parallel distraction and/or compression of vertebral tissue.

In some embodiments, the present surgical system can be employed with fracture reduction. In some embodiments, the present surgical system includes a fracture reduction system that employs surgical navigation and instrumentation to provide measurement capability. In some embodiments, the instrumentation includes fenestrated bone screws, for example, cement-augmented screws. In some embodiments, the fracture reduction system allows restoration of an angle during ligamentotaxy at a level of an implant, such as, for example, a head of a screw disposed at a surgical site to facilitate a more precise reduction of the trauma. In some embodiments, the fracture reduction system facilitates burst fracture reduction and/or deformity correction procedures. In some embodiment, to treat burst fractures, the fracture reduction system utilizes fenestrated bone screws including a sagittal adjusting screw (SAS), a fixed angle screw (FAS) and/or a multi-axial screw (MAS) for treating tumor, trauma and/or deformity disorders with instrumentation.

In some embodiments, the present surgical system includes a cement delivery system for fenestrated screws before and after a selected manipulation strategy including a procedure for correction. In some embodiments, the present surgical system includes a multi-level percutaneous fixation system and other features such as integrated extensions, and a low profile configuration. In some embodiments, the present surgical system includes a cement delivery system that delivers cement and/or other materials to one or more fenestrated screws to achieve various desired characteristics such as strength, rigidity, elasticity, compliance, biomechanical performance, durability and radiolucency or imaging preference on the implanted screws. In some embodiments, the present surgical system includes measurement capability that allows measurement and calculation of screw positions, relative correction and/or geometric parameters associated with the implanted screws. In some embodiments, the measurement capability is enabled by a computer through navigation software to quantify ligamentotaxy or kyphosis correction. In some embodiments, the present surgical system includes measurement capability that facilitates navigation of screws during a surgical procedure, which minimizes the exposure to radiation or other radioactive scanning devices.

In some embodiments, the present surgical system can be employed with a fracture reduction method including the steps of attaching a navigation reference marker with a patient anatomy, imaging the patient anatomy for registration of anatomical image data and positional tracking of the patient anatomy, implanting screws with driver that has a navigation component attached, imaging screws and vertebrae with a driver having a navigation component such that the navigation system projects and/or reproduces an image of the implant disposed with vertebrae on a computer screen. The image may include pre and post-correction orientation of the patient anatomy. In some embodiments, the method further includes the steps of selecting an implant strategy for at least one bone fastener; selecting a manipulation strategy for the patient anatomy prior or post the correction of vertebrae or other surgical process performed; a computer calculating a preferred correction based on screw orientation with vertebrae-pre correction; attaching a distractor to screws and/or employ other surgical instruments, selectively injecting cement with fractured vertebrae with a tool through fenestrated screws; performing correction with the distractor; imaging screws with vertebrae-post correction, comparing and/or evaluating calculated correction with actual correction on the navigation system.

In some embodiments, the present surgical system includes a surgical trauma instrument. In some embodiments, the present surgical system is utilized with a method to correct complex spinal deformities. In some embodiments, the present surgical system is utilized with a method to treat degenerative spinal disorders and/or employed with transforaminal lumbar interbody fusion procedures. In some embodiments, the present surgical system comprises a plurality of compressor/distractors, such as, for example, two distractors disposed along a side of vertebrae to perform a ligamentotaxy procedure. In some embodiments, the present surgical system comprises a single distractor to treat degenerative spinal disorders, for example, for disposal along a side of vertebrae oriented for decompression and/or interbody cage insertion.

In some embodiments, the present surgical system includes a percutaneous surgical system for use in a ligamentotaxy of a traumatic thoracic lumbar spine. In some embodiments, the present surgical system includes a surgical system that allows restoration of an angle during ligamentotaxy at a level of an implant, such as, for example, a head of a screw disposed at a surgical site to facilitate a more precise reduction of the trauma.

In some embodiments, the present surgical system includes a surgical instrument connected with an adaptor, which is utilized with a bone screw having extender tabs attached thereto. In some embodiments, the present surgical system includes an implant support including a connector and an adaptor. In some embodiments, the connector includes an outer sleeve configured for connection with extenders. In some embodiments, the connector is connected with extenders for insertion of an implant, such as for example, a spinal rod. In some embodiments, the adaptor includes an arm having a pivot hinge that connects the connector with a compressor/retractor. In some embodiments, the pivot hinge allows movement of the components to provide surgical-site visibility for inter-operative imaging. In some embodiments, a compressor/distractor is utilized for parallel distraction. In some embodiments, the surgical instrument includes a compressor/distractor having a reversible ratchet with a neutral, freely moveable position. In some embodiments, the present surgical system is employed with a procedure for implantation of a bone fastener percutaneously.

In some embodiments, the present surgical system includes a surgical instrument configured to compress or distract and restore curvature of a spine. In some embodiments, the present surgical system includes instruments and tools for correcting a sagittal deformity and rebalancing a spine of a body. In some embodiments, the present surgical system is employed to treat degenerative deformities of a spine in a sagittal plane, for example, degenerative kyphosis. In some embodiments, the present surgical system is employed to treat hyper-kyphosis, flat lumbar back, including disorders that create an unbalance of a body and loss of alignment between body parts. In some embodiments, the present surgical system provides a selected amount of correction to apply a selected balance to a spine and provides control and adjustment to the amount of correction. In some embodiments, the present surgical system includes a series of tools and instruments that allow formulation of a type of correction applied and can control the correction stabilization using posterior instrumentation.

In some embodiments, one or all of the components of the surgical system are disposable, peel-pack, pre-packed sterile devices used with a spinal construct. One or all of the components of the surgical system may be reusable. The surgical system may be configured as a kit with multiple sized and configured components.

In some embodiments, the present disclosure may be employed to treat spinal disorders such as, for example, degenerative disc disease, disc herniation, osteoporosis, spondylolisthesis, stenosis, scoliosis, kyphosis, and other curvature abnormalities, tumor and fractures. In some embodiments, the present disclosure may be employed with other osteo and bone related applications, including those associated with diagnostics and therapeutics. In some embodiments, the disclosed surgical system and methods may be alternatively employed in a surgical treatment with a patient in a prone or supine position, and/or employ various surgical approaches to the spine, including posterior and/or posterior mid-line and in other body regions. The present disclosure may also be alternatively employed with procedures for treating the lumbar, cervical, thoracic, sacral and pelvic regions of a spinal column. The system and methods of the present disclosure may also be used on animals, bone models and other non-living substrates, such as, for example, in training, testing and demonstration.

The present disclosure may be understood more readily by reference to the following detailed description of the embodiments taken in connection with the accompanying drawing figures, which form a part of this disclosure. It is to be understood that this application is not limited to the specific devices, methods, conditions or parameters described and/or shown herein, and that the terminology used herein is for the purpose of describing particular embodiments by way of example only and is not intended to be limiting. In some embodiments, as used in the specification and including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about," it will be understood that the particular value forms another embodiment. It is also understood that all spatial references, such as, for example, horizontal, vertical, top, upper, lower, bottom, left and right, are for illustrative purposes only and can be varied within the scope of the disclosure. For example, the references "upper" and "lower" are relative and used only in the context to the other, and are not necessarily "superior" and "inferior".

As used in the specification and including the appended claims, "treating" or "treatment" of a disease or condition refers to performing a procedure that may include administering one or more drugs to a patient (human, normal or otherwise or other mammal), employing implantable devices, and/or employing instruments that treat the disease, such as, for example, microdiscectomy instruments used to remove portions bulging or herniated discs and/or bone spurs, in an effort to alleviate signs or symptoms of the disease or condition. Alleviation can occur prior to signs or symptoms of the disease or condition appearing, as well as after their appearance. Thus, treating or treatment includes preventing or prevention of disease or undesirable condition (e.g., preventing the disease from occurring in a patient, who may be predisposed to the disease but has not yet been diagnosed as having it). In addition, treating or treatment does not require complete alleviation of signs or symptoms, does not require a cure, and specifically includes procedures that have only a marginal effect on the patient. Treatment can include inhibiting the disease, e.g., arresting its development, or relieving the disease, e.g., causing regression of the disease. For example, treatment can include reducing acute or chronic inflammation; alleviating pain and mitigating and inducing re-growth of new ligament, bone and other tissues; as an adjunct in surgery; and/or any repair procedure. Also, as used in the specification and including the appended claims, the term "tissue" includes soft tissue, ligaments, tendons, cartilage and/or bone unless specifically referred to otherwise.

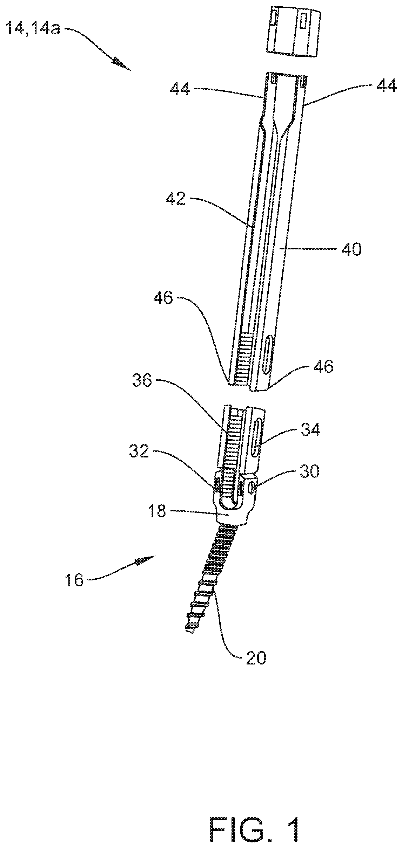

The following discussion includes a description of a surgical system and related methods of employing the surgical system in accordance with the principles of the present disclosure. Alternate embodiments are disclosed. Reference is made to the exemplary embodiments of the present disclosure, which are illustrated in the accompanying figures. Turning to FIGS. 1-3, there are illustrated components of a surgical system 10.

The components of surgical system 10 can be fabricated from biologically acceptable materials suitable for medical applications, including metals, synthetic polymers, ceramics and bone material and/or their composites. For example, the components of surgical system 10, individually or collectively, can be fabricated from materials such as stainless steel alloys, commercially pure titanium, titanium alloys, Grade 5 titanium, super-elastic titanium alloys, cobalt-chrome alloys, superelastic metallic alloys (e.g., Nitinol, super elasto-plastic metals), ceramics and composites thereof such as calcium phosphate (e.g., SKELITE^{™}), thermoplastics such as polyaryletherketone (PAEK) including polyetheretherketone (PEEK), polyetherketoneketone (PEKK) and polyetherketone (PEK), carbon-PEEK composites, PEEK-BaSO₄ polymeric rubbers, polyethylene terephthalate (PET), fabric, silicone, polyurethane, silicone-polyurethane copolymers, polymeric rubbers, polyolefin rubbers, hydrogels, semi-rigid and rigid materials, elastomers, rubbers, thermoplastic elastomers, thermoset elastomers, elastomeric composites, rigid polymers including polyphenylene, polyamide, polyimide, polyetherimide, polyethylene, epoxy, bone material including autograft, allograft, xenograft or transgenic cortical and/or corticocancellous bone, and tissue growth or differentiation factors, partially resorbable materials, such as, for example, composites of metals and calcium-based ceramics, composites of PEEK and calcium based ceramics, composites of PEEK with resorbable polymers, totally resorbable materials, such as, for example, calcium based ceramics such as calcium phosphate, tri-calcium phosphate (TCP), hydroxyapatite (HA)-TCP, calcium sulfate, or other resorbable polymers such as polyaetide, polyglycolide, polytyrosine carbonate, polycaroplaetohe and their combinations.

Various components of surgical system 10 may have material composites, including the above materials, to achieve various desired characteristics such as strength, rigidity, elasticity, compliance, biomechanical performance, durability and radiolucency or imaging preference. The components of surgical system 10, individually or collectively, may also be fabricated from a heterogeneous material such as a combination of two or more of the above-described materials. The components of surgical system 10 may be monolithically formed, integrally connected or include fastening elements and/or instruments, as described herein.

Surgical system 10 is employed, for example, with a minimally invasive procedure, including percutaneous techniques, mini-open and open surgical techniques to deliver and introduce instrumentation and/or components of spinal constructs at a surgical site within a body of a patient, for example, a section of a spine. In some embodiments, one or more of the components of surgical system 10 are configured for engagement with spinal constructs attached with vertebrae to manipulate tissue and/or correct a spinal disorder, such as, for example, a sagittal deformity, as described herein. In some embodiments, surgical system 10 may be employed with surgical procedures, such as, for example, corpectomy, discectomy and/or fracture/trauma treatment and may include fusion and/or fixation that employ implants to restore the mechanical support function of vertebrae.

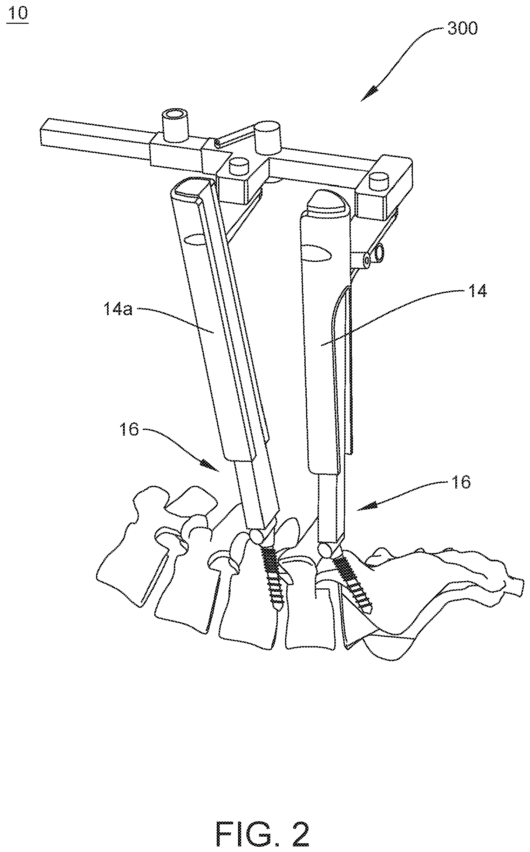

Surgical system 10 includes a surgical instrument, such as, for example, an implant support 14 and an implant support 14a, similar to implant support 14, as show in FIG. 2. Implant supports 14, 14a are each connectable with a bone fastener 16 having a receiver 18 and a screw shaft 20, as shown in FIGS. 1-3. Screw shaft 20 is fixed with tissue. Receiver 18 is connectable with implant support 14 to releasably engage a surgical instrument, such as, for example, a compressor/distractor 300 to distract and/or compress tissue.

Receiver 18 includes a pair of spaced apart arms 30, 32 that define an implant cavity configured for disposal of a component of a spinal construct, such as, for example, a spinal rod. Receiver 18 includes an inner surface having a thread form located disposed with arms, 30, 32. Screw shaft 20 is configured to penetrate tissue, such as, for example, bone, of a patient.

Arm 30 includes a break away tab 34 that is frangibly connected to arm 30 such that manipulation of tab 34 relative to arm 30 can fracture and separate tab 34 from arm 30 at a predetermined force and/or torque limit. Arm 32 includes a break away tab 36 that is frangibly connected to arm 32 such that manipulation of tab 36 relative to arm 32 can fracture and separate tab 36 from arm 32 at a predetermined force and/or torque limit, as described herein. In some embodiments, as force and/or torque is applied to tabs 34, 36 and resistance increases, for example, the predetermined torque and force limit is approached.

Implant support 14 includes extender tabs 40, 42 that are connectable with bone fastener 16, as shown in FIG. 1. Each extender tab 40, 42 extends between a proximal end 44 and a distal end 46. Distal ends 46 are configured for slidable disposal of a portion of bone fastener 16, such as, for example, tabs 34, 36. In some embodiments, tabs 34, 36 are configured to releasably fix extender tabs 40, 42 with bone fastener 16 for connection with implant support 14, as described herein. In some embodiments, bone fastener 16 includes at least one extender tab. For embodiments lacking tabs 34, 36, the extender tabs 40, 42 can connect directly to the arms 30, 32.

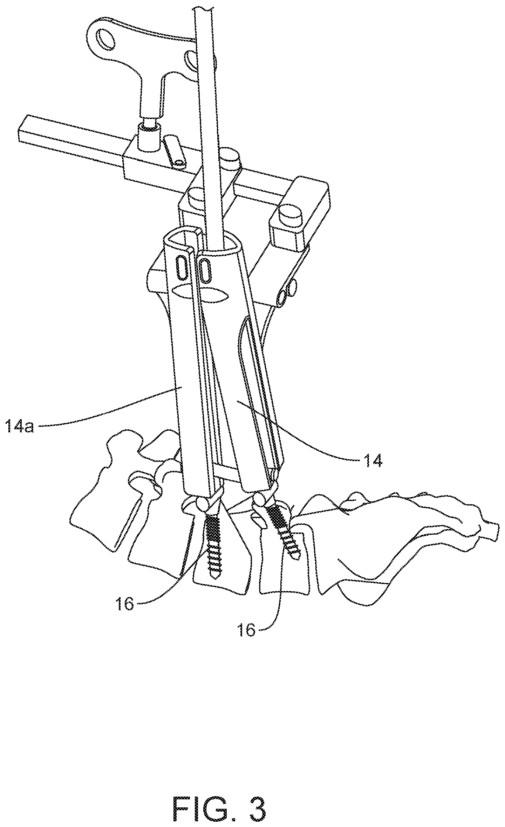

In some embodiments, connection of implant supports 14, 14a facilitates correction of a vertebral angle of vertebrae, for example, to achieve a selected lordosis and/or kyphosis, via manipulation of implant supports 14, 14a, as described herein. In some embodiments, implant supports 14, 14a are connected with compressor/distractor 300 to maintain a corrected vertebral angle of vertebrae during distraction and/or compression, as described herein. In some embodiments, the compressor/distractor has direct connection to extender (top-loading) and the screws such that the system provides direct control ligamentotaxy/kyphosis correction and at a reduced profile. In some embodiments, the present surgical system also provides ergonomic instrumentation that connects the implant, implant support and other rack and lordosis module assembly. In some embodiments, the present surgical system is configured for open and/or mini-open approach and configured as a pre-assembled single block top loading. In some embodiments, the surgical system includes an angulation module to facilitate compression/distraction. In some embodiments, an angulation module is configured for connection with the extenders 40, 42. For example, an angulation module may move the extenders close together in a compression process as shown in FIG. 3. In some embodiments, the angulation module is monolithic with the at least one implant support 14.

In assembly, operation and use, surgical system 10, similar to the systems and methods described herein, is employed with a surgical procedure, for treatment of a spine of a patient including vertebrae V. Surgical system 10 may also be employed with surgical procedures, such as, for example, discectomy, laminectomy, fusion, laminotomy, laminectomy, nerve root retraction, foramenotomy, facetectomy, decompression, spinal nucleus or disc replacement and bone graft and implantable prosthetics including plates, rods, and bone engaging fasteners.

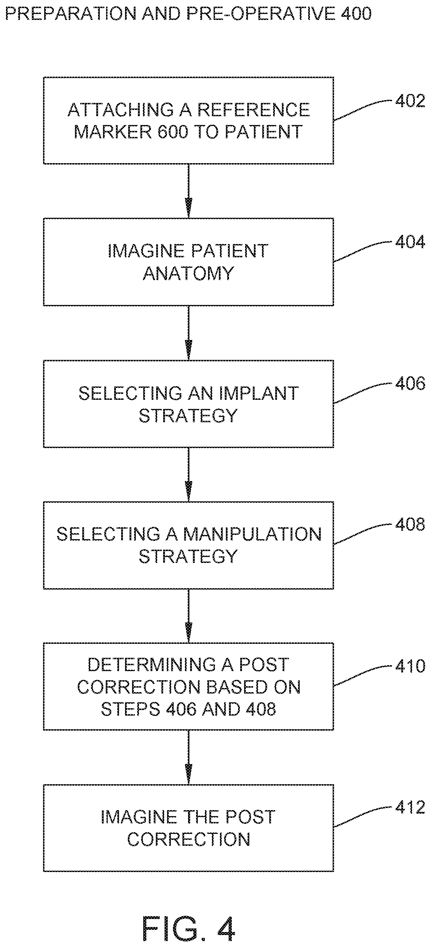

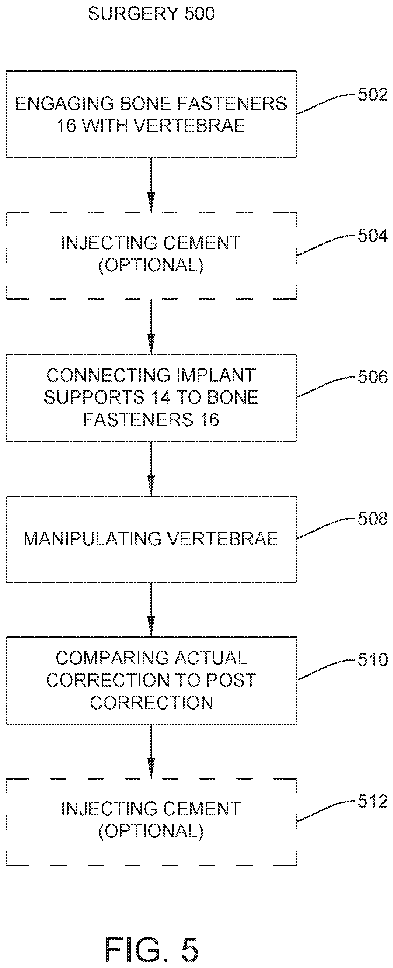

In various embodiment, as shown by way of examples in FIGS. 4-12, spinal implant system 10, similar to the systems and methods described herein, is employed in connection with one or more surgical procedures, as described herein. Initially, spinal implant system 10 is employed with a method for treating a spine that includes a preparation and/or a pre-operative procedure 400, as shown in FIG. 4. Surgical method 500, as shown in FIG. 5, includes use of components and/or surgical instruments for delivery of components of spinal implant system 10, which, such as, for example, drivers, extenders, reducers, spreaders, distractors, blades, clamps, forceps, elevators and drills, any of which may be alternately sized and dimensioned, and any of which may be arranged as a kit.

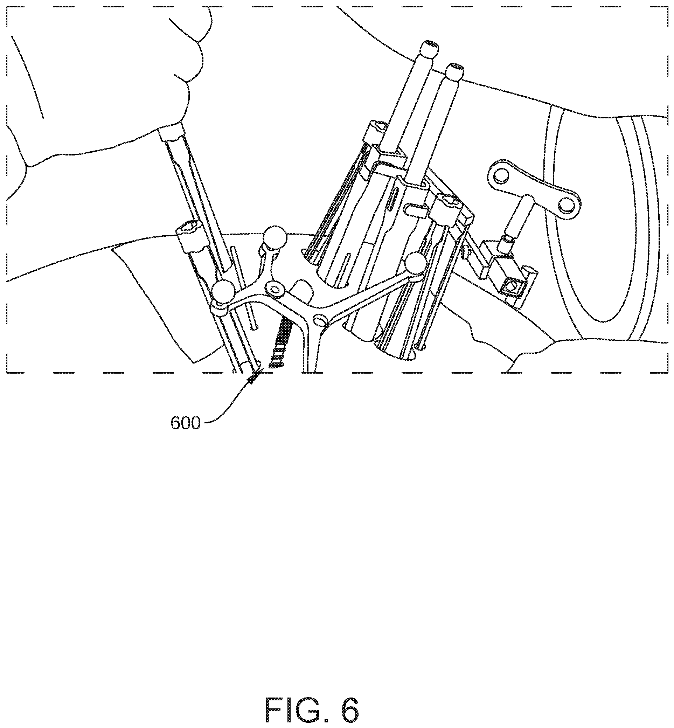

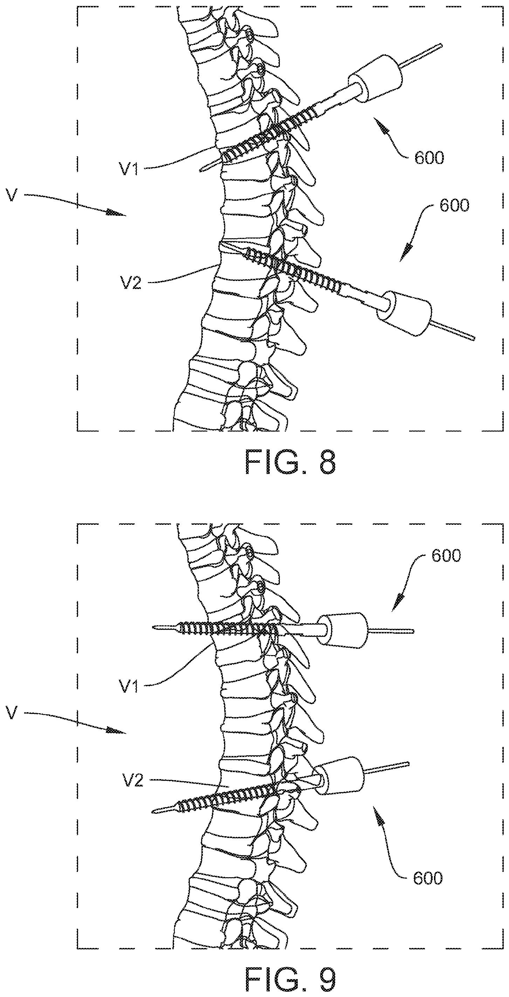

In preparation and/or a pre-operative procedure 400, step 402 includes attaching a 3D reference marker 600, as shown in FIG. 6, to the patient. Marker 600 is visible by surgical navigation system 216, shown in FIGS. 8 and 9. Marker 600 is utilized to obtain images bone fastener 16 in a coronal, cephalad and/or caudal space during angulation, distraction and/or derotation. In some embodiments, markers 600 can be utilized with two or a plurality of bone fasteners 16 at multiple vertebral levels. For example, prior to manipulation of the vertebral segment, a machine can overlay bone fasteners 16 to vertebrae V.

Step 404 includes imaging vertebrae V for registration of anatomical image data and positional tracking of vertebrae V, for example, a CT scan or MRI scan. In some embodiments, the images include taking C-arm images of the patient anatomy. The images are calibrated such that a three-dimensional image of the selected vertebrae is generated. In some embodiments, fluoroscopic images are taken from different angles, such as, 0, 45, and 90 degrees. In some embodiments, multiple C-arm images are taken. For example, step 404 may include imaging the vertebrae V by performing a three-dimensional scan of the selected vertebrae V. In some embodiments, step 404 includes utilizing O-Arm^{®} imaging device in the operating room to obtain the three-dimensional images of the patient anatomy intra-operatively and/or prior to surgery 500 for calibration and registration.

During a registration process, the three-dimensional scans from step 404 are transferred to a control unit. See, for example, the registration systems and methods, as described in U.S. Patent No. 8,571,638.

The C-arm images from step 404 are transferred to the control unit. A pseudo three-dimensional image of the selected vertebrae V is generated for patient anatomy registration, FIG. 8. For example, at pre-correction, the relative angle between a first bone fastener 16 is about 51 degrees relative to a second bone fastener 16 and a distance is about 41 mm.

In step 406, a surgeon reviews three-dimensional the images captured in step 404 and formulates and selects an implant strategy according to three-dimensional scans.

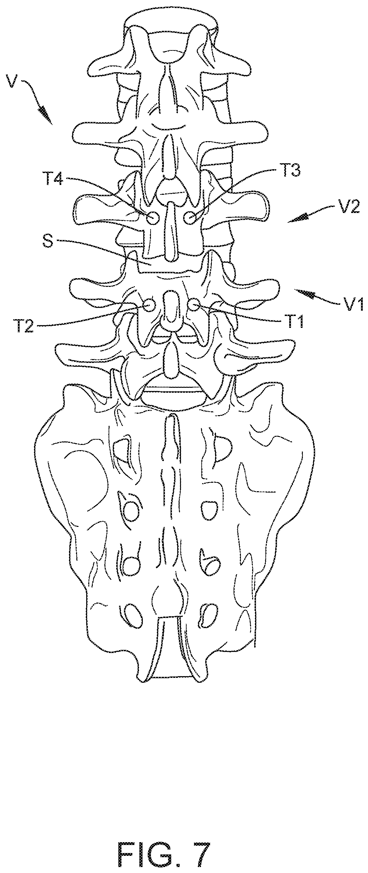

Step 406 includes selecting an implant strategy for bone fasteners 16. For example, step 406 includes selecting a plurality of trajectories, such as, for example, trajectories T1, T2, T3 and T4 for bone fasteners 16 for positioning and engaging with vertebrae V, as shown in FIG. 7. In some embodiments, step 406 utilizes pre-operative analytics software including anatomy recognition and vertebral segmentation algorithms for surgical visualization based on a patient's images, which facilitates formulating the implant strategy including implant and trajectory placement planning.

Step 408 includes selecting a manipulation strategy for bone fasteners 16. Step 408 includes forming a strategy for manipulating a first bone fastener 16 a selected angular orientation relative to a second bone fastener 16 to decompress the vertebral tissue of vertebrae V1, V2.

Step 410 includes determining a post-correction orientation of vertebrae V1, V2 according to the implant strategy from step 406 and the manipulation strategy from step 408. In various embodiments, step 410 includes calculating or otherwise determining the post-correction orientation using a specially configured computing system having a processor. In various embodiments, calculating the post-correction orientation is done by a specially configured computing device (e.g., a processor executing software customized for the present purpose).

Step 412 includes imaging the calculated post-correction orientation, as shown in FIG. 9. For example, the calculated post correction would manipulate bone fasteners 16 such that the relative angle between the first bone fastener 16 is about 7 degrees relative to the second bone fastener 16 and a distance is about 27 mm.

In contemplated embodiments, any of the steps of the methods described herein can be performed by a specially configured computing system - e.g., a processor executing instructions, stored on a tangible, non-transitory, computer-readable storage device, customized for the present purpose.

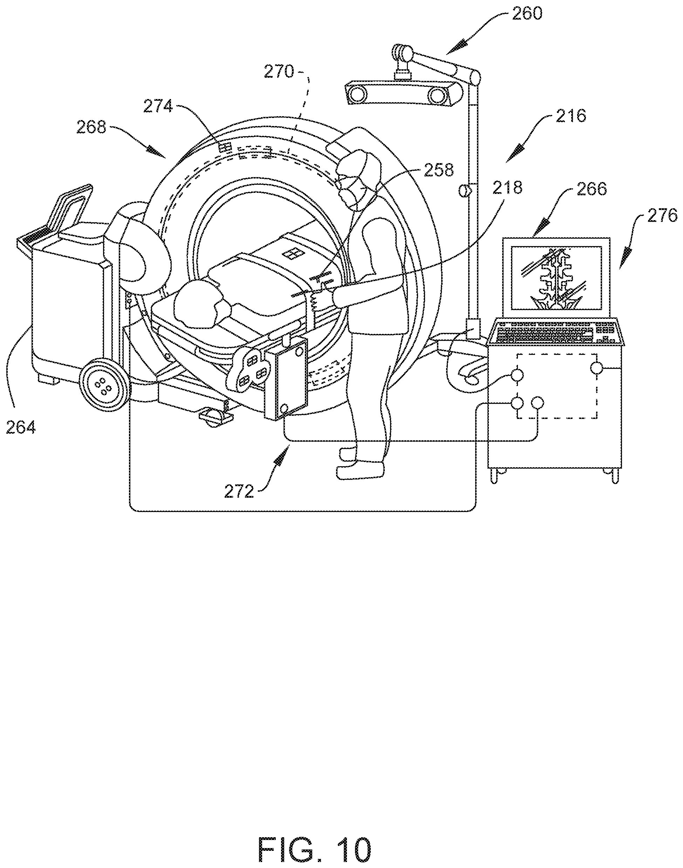

During surgery 500, step 502 includes engaging bone fasteners 16 with vertebrae V1, V2 (see e.g., FIGS. 8 and 9) along trajectories T1, T2, T3 and T4 (see e.g., FIG. 7) according to the implant strategy developed in step 406. Bone fasteners 16 are connected with a surgical instrument, such as, for example, a driver 218 including a navigation component 258, as shown in FIG. 10. Navigation component 258 generates a signal representative of a position of each bone fastener 16 relative to vertebrae V1, V3 based on the actual correction orientation.

Navigation component 258 transmits a signal and communicates with a processor of computer 264 of a navigation system 216, as shown in FIG. 10. In various embodiments, navigation component 258 includes an emitter array. The emitter array is configured for generating a signal to a sensor array 260 of surgical navigation system 16. In some embodiments, the signal generated by emitter array 262 represents a position of bone fastener 16 relative to driver 218 and relative to tissue, such as, for example, bone. In some embodiments, the signal generated by emitter array 262 represents a three-dimensional position of bone fastener 16 relative to tissue.

In some embodiments, sensor array 260 receives signals from emitter array 262 to provide a three-dimensional spatial position and/or a trajectory of bone fastener 16 relative to driver 218 and/or tissue. Emitter array 262 communicates with a processor of computer 264 of navigation system 216 to generate data for display of an image on a monitor 266. In some embodiments, sensor array 260 receives signals from emitter array 262 to provide a visual representation of a position of bone fastener 16 relative to driver 218 and/or tissue. See, for example, similar surgical navigation components and their use as described in U.S. Patent Nos. 6,021,343, 6,725,080, 6,796,988.

Surgical navigation system 216 is configured for acquiring and displaying medical imaging, such as, for example, x-ray images appropriate for a given surgical procedure. In some embodiments, pre-acquired images of a patient are collected. In some embodiments, surgical navigation system 216 can include an O-arm^{®} imaging device 268 sold by Medtronic Navigation, Inc., having a place of business in Louisville, Colo., USA. Imaging device 68 may have a generally annular gantry housing that encloses an image capturing portion 270.

In some embodiments, image capturing portion 270 includes an x-ray source or emission portion and an x-ray-receiving, or other image-receiving, portion located generally, or as practically possible, 180 degrees from each other and mounted on a rotor (not shown in detail) relative to a track of image-capturing portion 270. Image-capturing portion 270 can be operable to rotate 360 degrees during image acquisition. Image-capturing portion 270 may rotate around a central point or axis, allowing image data of the patient to be acquired from multiple directions or in multiple planes. Surgical navigation system 216 can include those disclosed in U.S. Pat. Nos. 8,842,893, 7,188,998; 7,108,421; 7,106,825; 7,001,045; and 6,940,941.

The images, e.g., fluoroscopic images, taken are transmitted to computer 264, which may further forward them toto a computer 276. Image transfer may be performed using any channel, wireless or wired, such as over a standard video connection or a digital link including wired and wireless. In various embodiments, computer 276 provides the ability to display, via monitor 266, as well as save, digitally manipulate, or print a hard copy of the received images. In some embodiments, images may also be displayed to the surgeon through a heads-up display. In a contemplated embodiment, one computer is employed, here, instead of the two computers 264, 276.

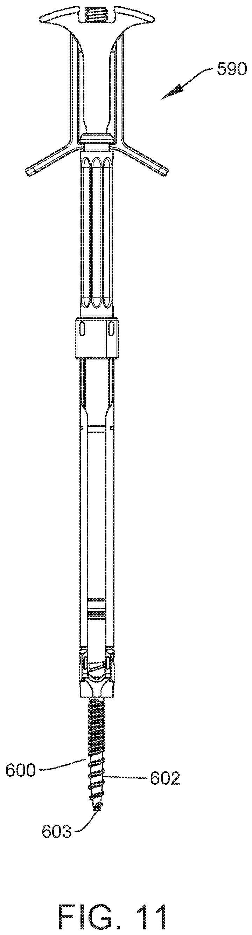

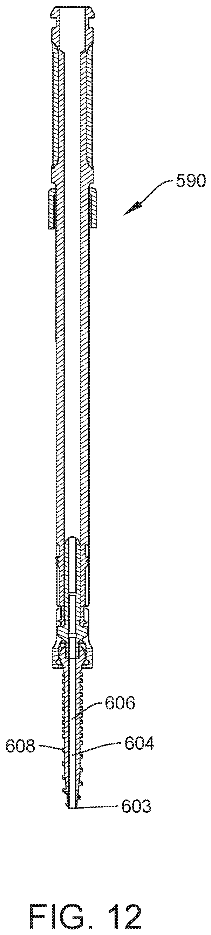

In some embodiments, in step 504 a surgical instrument, such as, for example, a cement delivery device 590, as shown in FIGS. 11 and 12, is connected with bone fasteners 16 to inject cement through bone fasteners 16 into vertebrae V1, V2. In some embodiments, bone fasteners 16 includes fenestrated bone fasteners 160, as shown in FIGS. 11 and 12. In some embodiments, bone fastener 600 includes a shaft 602 including a proximal portion and a closed distal tip 603. Shaft 602 includes an outer surface engageable with a first cortical surface and a second cortical surface of bone. The proximal portion includes an inner surface 604 that defines a longitudinal cavity 606. Shaft 602 includes at least one opening, such as, for example, a fenestration 608 in communication with cavity 606 for dispersing the cement into vertebrae V. In some embodiments, bone fasteners 16 include sagittally adjustable screws. The bone fasteners 16 can in any way be like those described in U.S. Pat. Ser. No. 16/047,590**.**

In step 506 implant supports 14, 14a are connected with bone fasteners 16. For example, implant supports 14, 14a are connected with extenders 40, 42, as described herein.

In step 508, compressor/distractor 300 is mounted with adaptors 100, as described herein, to allow for manipulation via distraction and/or compression of vertebrae V1, V2 for the actual correction based on the manipulation strategy developed at step 408.

Step 510 includes comparing the actual correction from step 508 of vertebrae V1, V2 with the post-correction determined in steps 410 and 412.

In some embodiments, in step 512, cement delivery device 590 is connected with bone fasteners 160, as described herein, to inject cement through bone fasteners 160 into vertebral space S of vertebrae V1, V2.

Upon completion of one or more surgical procedures, the surgical instruments and non-implanted components of spinal implant system 10 are removed and the incision(s) are closed. One or more of the components of spinal implant system 10 can be made of radiolucent materials such as polymers. In some embodiments, spinal implant system 10 includes an agent, which may be disposed, packed, coated or layered within, on or about the components and/or surfaces of spinal implant system 10. In some embodiments, the agent may include bone growth promoting material, such as, for example, bone graft to enhance fixation of the fixation elements with vertebrae. In some embodiments, the agent may be HA coating. In some embodiments, the agent may include one or a plurality of therapeutic agents and/or pharmacological agents for release, including sustained release, to treat, for example, pain, inflammation and degeneration.

It will be understood that various modifications may be made to the embodiments disclosed herein. Therefore, the above description should not be construed as limiting, but merely as exemplification of the various embodiments. Those skilled in the art will envision other modifications within the scope of the claims appended hereto.

## Claims

1. A spinal implant system (10) comprising:
a tangible storage device comprising computer-readable instructions;
a 3D reference marker (600), configured to be attached to a patient anatomy and configured to be oriented relative to a sensor (260) for registration of anatomical image data and positional tracking of the patient anatomy;
a tracking device including the sensor (260) that receives a signal and communicates with a processor to generate data for display of an image on a monitor (266), the image representing position of the 3D reference marker (600) relative to tissue; and
the processor, wherein the instructions are customized so that the processor executing the instructions in operation of the system (10) causes the spinal implant system (10) to perform:
imaging, pre-operatively, the patient anatomy with the 3D reference marker (600) attached to the patient anatomy for registration of anatomical image data and positional tracking of the patient anatomy, wherein imaging the patient anatomy includes performing a three-dimensional scan of selected vertebrae of the patient anatomy, and, pre-operatively, transferring the three-dimensional scan during a registration process to a control unit, to thereby allow a surgeon to determine, pre-operatively, an implant strategy for at least one bone fastener (16) as well as to determine, pre-operatively, based on the patient anatomy and the implant strategy, a manipulation strategy for a bone fastener (16) connected to an implant support (14);
determining, pre-operatively, a post-correction orientation of the patient anatomy according to the implant strategy and the manipulation strategy; and
displaying, pre-operatively, the post-correction orientation of the patient anatomy, wherein the instructions are customized further so that the processor further executing the instructions in operation of the spinal implant system (10) causes the spinal implant system to perform:
comparing, intra-operatively, an actual correction orientation of the patient anatomy according to the manipulation strategy with the pre-operatively determined post-correction orientation, wherein the spinal implant system (10) further comprises:
at least one bone fastener (16) having a screw shaft (20),
wherein determining, pre-operatively, the implant strategy includes selecting a screw trajectory for the screw shaft (20) of the bone fastener (16) connecting the screw shaft (20) to vertebral tissue.

2. Spinal implant system (10) as recited in Claim 1, wherein the at least one bone fastener (16) includes a plurality of pedicle screws and the implant strategy includes a corresponding plurality of respective screw trajectories for positioning the screws with a first vertebra (V1) and a second vertebra (V2) of the patient anatomy.

3. Spinal implant system (10) as recited in Claim 1, wherein the at least one bone fastener (16) includes a first bone screw (16) configured to be disposed with a first vertebra (V1) of the patient anatomy and a second bone screw (16) configured to be disposed with a second vertebra (V2) of the patient anatomy, and the manipulation strategy includes manipulating the first bone screw (16) a selected angular orientation relative to the second bone screw (16).

4. Spinal implant system as recited in Claim 1,
wherein the at least one bone fastener (16) includes a first bone fastener (16) and a second bone fastener (16), and
wherein the manipulation strategy includes manipulating the first bone fastener (16) a selected distance relative to a second bone fastener (16).

5. Spinal implant system (10) as recited in any one of Claims 1 to 4, wherein the at least one bone fastener (16) includes a fenestrated screw.

6. Spinal implant system (10) as recited in any one of Claims 2 to 4, wherein the at least one bone fastener (16) includes a sagittally adjustable screw.

7. Spinal implant system (10) as recited in any one of Claims 1 to 6, further comprising:
a surgical driver having a navigation component,
wherein the at least one bone fastener (16) is connected with the surgical driver, and the comparing includes the navigation component of the surgical driver generating a signal representative of a position of the at least one bone fastener (16).

8. Spinal implant system (10) as recited in any one of Claims 1 to 7, further comprising:
a compressor/distractor (300) configured to distract and/or compress vertebral tissue according to the manipulation strategy, the compressor/distractor (300) being mounted with an adaptor, and
the implant support (14), which is connected to the at least one bone fastener (16) and connected with the compressor/distractor (300), wherein the compressor/distractor (300) is releasably engaged to the adaptor that is movable relative to the implant support (14).

9. Spinal implant system (10) as recited in Claim 8,
wherein the compressor/distractor (300) includes a first member and a second member movable relative to the first member, the compressor/distractor (300) further includes a ratchet preventing movement of the second member relative to the first member in a first direction and a second direction.

## Patentansprüche

1. Wirbelsäulenimplantatsystem (10), umfassend:
eine materielle Speichervorrichtung umfassend computerlesbare Anweisungen;
einen 3D-Referenzmarker (600), der konfiguriert ist, um an einer Patientenanatomie angebracht zu werden, und konfiguriert ist, um relativ zu einem Sensor (260) ausgerichtet zu werden, für Registrierung anatomischer Bilddaten und Positionsverfolgung der Patientenanatomie;
eine Verfolgungsvorrichtung, einschließlich des Sensors (260), die ein Signal empfängt und mit einem Prozessor kommuniziert, um Daten für Anzeige eines Bilds auf einem Monitor (266) zu erzeugen, wobei das Bild eine Position des 3D-Referenzmarkers (600) relativ zu Gewebe darstellt; und
den Prozessor, wobei die Anweisungen angepasst sind, sodass der Prozessor, der die Anweisungen in einem Betrieb des Systems (10) ausführt, das Wirbelsäulenimplantatsystem (10) veranlasst, durchzuführen:
Bildgebung, präoperativ, der Patientenanatomie mit dem 3D-Referenzmarker (600), der an der Patientenanatomie angebracht ist, für Registrierung anatomischer Bilddaten und Positionsverfolgung der Patientenanatomie, wobei die Bildgebung der Patientenanatomie das Durchführen eines dreidimensionalen Scans ausgewählter Wirbel der Patientenanatomie und, präoperativ, ein Transferieren des dreidimensionalen Scans während eines Registrierungsverfahrens an eine Steuereinheit einschließt, um dadurch einem Chirurgen zu ermöglichen, präoperativ, eine Implantatstrategie für mindestens ein Knochenbefestigungselement (16) zu bestimmen sowie, präoperativ, basierend auf der Patientenanatomie und der Implantatstrategie, eine Manipulationsstrategie für ein Knochenbefestigungselement (16), das mit einem Implantatträger (14) verbunden ist, zu bestimmen;
Bestimmen, präoperativ, einer Postkorrekturausrichtung der Patientenanatomie gemäß der Implantatstrategie und der Manipulationsstrategie; und
Anzeigen, präoperativ, der Postkorrekturausrichtung der Patientenanatomie, wobei die Anweisungen ferner angepasst sind, sodass der Prozessor, der die Anweisungen in dem Betrieb des Wirbelsäulenimplantatsystems (10) ferner ausführt, das Wirbelsäulenimplantatsystem veranlasst, durchzuführen:
Vergleichen, intraoperativ, einer tatsächlichen Korrekturausrichtung der Patientenanatomie gemäß der Manipulationsstrategie mit der präoperativ bestimmten Postkorrekturausrichtung, wobei das Wirbelsäulenimplantatsystem (10) ferner umfasst:
mindestens ein Knochenbefestigungselement (16), das einen Schraubenschaft (20) aufweist,
wobei, das Bestimmen, präoperativ, der Implantatstrategie ein Auswählen einer Schraubentrajektorie für den Schraubenschaft (20) des Knochenbefestigungselements (16), die den Schraubenschaft (20) mit Wirbelgewebe verbindet, einschließt.

2. Wirbelsäulenimplantatsystem (10) nach Anspruch 1, wobei das mindestens eine Knochenbefestigungselement (16) eine Vielzahl von Pedikelschrauben einschließt und die Implantatstrategie eine entsprechende Vielzahl von jeweiligen Schraubentrajektorien zum Positionieren der Schrauben mit einem ersten Wirbel (V1) und einem zweiten Wirbel (V2) der Patientenanatomie einschließt.

3. Wirbelsäulenimplantatsystem (10) nach Anspruch 1, wobei das mindestens eine Knochenbefestigungselement (16) eine erste Knochenschraube (16), die konfiguriert ist, um an einem ersten Wirbel (V1) der Patientenanatomie angeordnet zu werden, und eine zweite Knochenschraube (16), die konfiguriert ist, um an einem zweiten Wirbel (V2) der Patientenanatomie angeordnet zu werden, einschließt, und die Manipulationsstrategie ein Manipulieren der ersten Knochenschraube (16) in eine ausgewählte Winkelausrichtung relativ zu der zweiten Knochenschraube (16) einschließt.

4. Wirbelsäulenimplantatsystem nach Anspruch 1,
wobei das mindestens eine Knochenbefestigungselement (16) ein erstes Knochenbefestigungselement (16) und ein zweites Knochenbefestigungselement (16) einschließt, und
wobei die Manipulationsstrategie das Manipulieren des ersten Knochenbefestigungselements (16) um einen ausgewählten Abstand relativ zu einem zweiten Knochenbefestigungselement (16) einschließt.

5. Wirbelsäulenimplantatsystem (10) nach einem der Ansprüche 1 bis 4, wobei das mindestens eine Knochenbefestigungselement (16) eine gefensterte Schraube einschließt.

6. Wirbelsäulenimplantatsystem (10) nach einem der Ansprüche 2 bis 4, wobei das mindestens eine Knochenbefestigungselement (16) eine sagittal einstellbare Schraube einschließt.

7. Wirbelsäulenimplantatsystem (10) nach einem der Ansprüche 1 bis 6, ferner umfassend:
einen chirurgischen Treiber, der eine Navigationskomponente aufweist,
wobei das mindestens eine Knochenbefestigungselement (16) mit dem chirurgischen Treiber verbunden ist und das Vergleichen einschließt, dass die Navigationskomponente des chirurgischen Treibers ein Signal, das eine Position des mindestens einen Knochenbefestigungselements (16) darstellt, erzeugt.

8. Wirbelsäulenimplantat (10) nach einem der Ansprüche 1 bis 7,
ferner umfassend:
einen Kompressor/Distraktor (300), der konfiguriert ist, um Wirbelgewebe gemäß der Manipulationsstrategie zu distrahieren und/oder zu komprimieren, wobei der Kompressor/Distraktor (300) mit einem Adapter montiert ist, und
den Implantatträger (14), der mit dem mindestens einen Knochenbefestigungselement (16) verbunden ist und mit dem Kompressor/Distraktor (300) verbunden ist, wobei der Kompressor/Distraktor (300) mit dem Adapter, der relativ zu dem Implantatträger (14) bewegbar ist, lösbar in Eingriff genommen ist.

9. Wirbelsäulenimplantatsystem (10) nach Anspruch 8,
wobei der Kompressor/Distraktor (300) ein erstes Teil und ein zweites Teil, das relativ zu dem ersten Teil bewegbar ist, einschließt, wobei der Kompressor/Distraktor (300) ferner ein Gesperre, das die Bewegung des zweiten Teils relativ zu dem ersten Teil in eine erste Richtung und eine zweite Richtung verhindert, einschließt.

## Revendications

1. Système d'implant rachidien (10) comprenant :
un dispositif de stockage tangible comprenant des instructions lisibles par ordinateur ;
un marqueur de référence 3D (600), conçu pour être fixé à l'anatomie d'un patient et conçu pour être orienté par rapport à un capteur (260) pour l'enregistrement de données d'imagerie anatomique et le suivi positionnel de l'anatomie du patient ;
un dispositif de suivi comportant le capteur (260) qui reçoit un signal et communique avec un processeur pour générer des données pour l'affichage d'une image sur un moniteur (266), l'image représentant la position du marqueur de référence 3D (600) par rapport au tissu ; et
le processeur, dans lequel les instructions sont personnalisées de sorte que le processeur exécutant les instructions en opération du système (10) amène le système d'implant rachidien (10) à réaliser :
l'imagerie, de manière préopératoire, de l'anatomie du patient avec le marqueur de référence 3D (600) fixé à l'anatomie du patient pour l'enregistrement de données d'imagerie anatomique et le suivi positionnel de l'anatomie du patient, dans lequel l'imagerie de l'anatomie du patient comporte la réalisation d'un balayage tridimensionnel de vertèbres sélectionnées de l'anatomie du patient et, de manière préopératoire, le transfert du balayage tridimensionnel pendant un processus d'enregistrement à une unité de commande, pour permettre de ce fait à un chirurgien de déterminer, de manière préopératoire, une stratégie d'implant pour au moins un élément d'ancrage osseux (16) ainsi que de déterminer, de manière préopératoire, en fonction de l'anatomie du patient et de la stratégie d'implant, une stratégie de manipulation pour un élément d'ancrage osseux (16) relié à un support d'implant (14) ;
la détermination, de manière préopératoire, d'une orientation post-correction de l'anatomie du patient selon la stratégie d'implant et la stratégie de manipulation ; et
l'affichage, de manière préopératoire, de l'orientation post-correction de l'anatomie du patient, dans lequel les instructions sont en outre personnalisées de sorte que le processeur exécutant en outre les instructions en opération du système d'implant rachidien (10) amène le système d'implant rachidien à réaliser :
la comparaison, de manière intraopératoire, d'une orientation de correction réelle de l'anatomie du patient selon la stratégie de manipulation avec l'orientation post-correction déterminée de manière préopératoire, dans lequel le système d'implant rachidien (10) comprend en outre :
au moins un élément d'ancrage osseux (16) ayant une tige de vis (20),
dans lequel la détermination, de manière préopératoire, de la stratégie d'implant comporte la sélection d'une trajectoire de vis pour la tige de vis (20) de l'élément d'ancrage osseux (16) reliant la tige de vis (20) au tissu vertébral.

2. Système d'implant rachidien (10) selon la revendication 1, dans lequel l'au moins un élément d'ancrage osseux (16) comporte une pluralité de vis pédiculaires et la stratégie d'implant comporte une pluralité correspondante de trajectoires de vis respectives permettant de positionner les vis avec une première vertèbre (V1) et une seconde vertèbre (V2) de l'anatomie du patient.

3. Système d'implant rachidien (10) selon la revendication 1, dans lequel l'au moins un élément d'ancrage osseux (16) comporte une première vis osseuse (16) conçue pour être disposée avec une première vertèbre (V1) de l'anatomie du patient et une seconde vis osseuse (16) conçue pour être disposée avec une seconde vertèbre (V2) de l'anatomie du patient, et la stratégie de manipulation comporte la manipulation de la première vis osseuse (16) selon une orientation angulaire sélectionnée par rapport à la seconde vis osseuse (16).

4. Système d'implant rachidien selon la revendication 1,
dans lequel l'au moins un élément d'ancrage osseux (16) comporte un premier élément d'ancrage osseux (16) et un second élément d'ancrage osseux (16), et
dans lequel la stratégie de manipulation comporte la manipulation du premier élément d'ancrage osseux (16) à une distance sélectionnée par rapport à un second élément d'ancrage osseux (16).

5. Système d'implant rachidien (10) selon l'une quelconque des revendications 1 à 4, dans lequel l'au moins un élément d'ancrage osseux (16) comporte une vis fenêtrée.

6. Système d'implant rachidien (10) selon l'une quelconque des revendications 2 à 4, dans lequel l'au moins un élément d'ancrage osseux (16) comporte une vis réglable de manière sagittale.

7. Système d'implant rachidien (10) selon l'une quelconque des revendications 1 à 6, comprenant en outre :
un tournevis chirurgical ayant un composant de navigation,
dans lequel l'au moins un élément d'ancrage osseux (16) est relié au tournevis chirurgical, et la comparaison comporte le composant de navigation du tournevis chirurgical générant un signal représentant une position de l'au moins un élément d'ancrage osseux (16).

8. Système d'implant rachidien (10) selon l'une quelconque des revendications 1 à 7,
comprenant en outre :
un compresseur/distracteur (300) conçu pour distracter et/ou comprimer le tissu vertébral selon la stratégie de manipulation, le compresseur/distracteur (300) étant monté avec un adaptateur, et
le support d'implant (14), qui est relié à l'au moins un élément d'ancrage osseux (16) et relié au compresseur/distracteur (300), dans lequel le compresseur/distracteur (300) est en prise de manière libérable avec l'adaptateur qui est mobile par rapport au support d'implant (14).

9. Système d'implant rachidien (10) selon la revendication 8,
dans lequel le compresseur/distracteur (300) comporte un premier organe et un second organe mobile par rapport au premier organe, le compresseur/distracteur (300) comporte en outre un cliquet empêchant un mouvement du second organe par rapport au premier organe dans un premier sens et un second sens.
